# EUROPÄISCHE PATENTSCHRIFT

(11) **EP 2 286 747 B1**
(45) Veröffentlichungstag und Bekanntmachung des Hinweises auf die Patenterteilung: **08.04.2015**
(21) Anmeldenummer: 10186228.2
(22) Anmeldetag: 23.02.2005
(51) Int. Cl.: A61B 17/70

(54) **Verankerungselement und Einrichtung zur dynamischen Stabilisierung von Wirbeln bzw. Knochen**
Enchoring element and dynamic stabilisation device for vertebral bodies or bones
Elément d'ancrage et dispositif de stabilisation dynamique pour vertèbres ou os.

(30) Priorität: 03.03.2004 DE 102004010380; 03.03.2004 US 550008 P
(43) Veröffentlichungstag der Anmeldung: 23.02.2011
(62) Teilanmeldung aus: 05003913.0
(73) Patentinhaber: Biedermann Technologies GmbH & Co. KG, 78166 Donaueschingen (DE)
(72) Erfinder: Harms, Jürgen, 76227, Karlsruhe (DE); Matthis, Wilfried, 79367, Weisweil (DE); Biedermann, Lutz, 78048, VS-Villingen (DE)
(74) Vertreter: Prüfer & Partner GbR European Patent Attorneys

(56) Entgegenhaltungen:
- US-A- 4 946 458
- US-A1- 2002 198 527
- US-A1- 2003 045 879
- US-B1- 6 368 321
- US-B1- 6 471 705

## Beschreibung

Die Erfindung betrifft ein Verankerungselement und eine Stabilisierungseinrichtung zur dynamischen Stabilisierung von Wirbeln oder Knochen mit einem solchen Verankerungselement.

Zur Fixierung von Knochenfrakturen oder zur Stabilisierung der Wirbelsäule sind starre Fixations- und Stabilisierungseinrichtungen bekannt, die aus wenigstens zwei jeweils in einem Knochen bzw. Wirbel zu verankernden und über einen starren Stab miteinander verbundenen Knochenschrauben bestehen. Beispielsweise ist aus der EP 0 483 242 ein Verankerungselement nach dem Oberbegriff des Patentanspruchs 1 bekannt, das zusammen mit einem starren Stab für eine solche starre Stabilisierungseinrichtung verwendet wird. Starre Systeme werden dann eingesetzt, wenn eine Relativbewegung der zu stabilisierenden Knochenteile oder Wirbel zueinander nicht erwünscht ist, z.B. beim Vorhandensein von Frakturen oder anderen Fehlstellungen.

Aus der US 5,474,555 ist ein Knochenverankerungselement in Form einer Polyaxial-Knochenschraube mit einem Schraubenelement und einem Aufnahmeteil zur Verbindung mit einem Stab bekannt, bei der das im Knochen zu verankernde Schraubenelement mit dem Aufnahmeteil derart verbunden ist, dass eine begrenzte Bewegung zwischen dem Schraubenelement und dem Aufnahmeteil möglich ist. Die beschriebene Lösung erlaubt jedoch keine Stabilisierung mit einer kontrollierten Bewegungsmöglichkeit.

Bei bestimmten Indikationen, z.B. bei einer geschädigten Bandscheibe oder bei Vorhandensein einer künstlichen Bandscheibe ist eine Stabilisierungseinrichtung wünschenswert, die eine begrenzte Bewegung der zu stabilisierenden Wirbel ermöglicht. Eine derartige dynamische Stabilisierungseinrichtung ist beispielsweise aus der US 5,733,284 bekannt.

Bei den bekannten Stabilisierungsvorrichtungen, insbesondere den dynamischen, besteht die Gefahr, dass über den Stab ein Drehmoment auf das Verankerungselement ausgeübt wird. Dies kann dazu führen, dass es zu einem Lockern oder gar Lösen des Verankerungselementes im Knochen kommt.

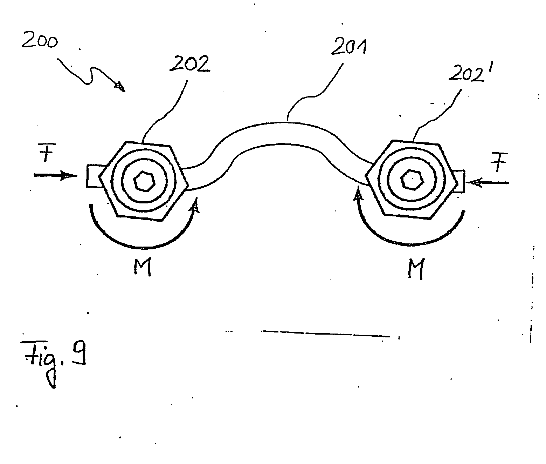

In Fig. 9 ist das Zustandekommen eines Drehmomentes M um die Schraubenachse bei einer der Anmelderin bekannten dynamischen Stabilisierungseinrichtung 200 dargestellt. Bei der hier dargestellten Stabilisierungseinrichtung 200 sind zwei Knochenverankerungselemente 202, 202' über einen gekrümmten Stab 201 mit einer vorbestimmten Biege-Elastizität miteinander verbunden. Die Knochenverankerungselemente 202, 202' sind mit Knochenschrauben fest in zwei benachbarten Wirbeln (hier nicht dargestellt) verankert. In Fig. 9 ist der Fall dargestellt, dass die beiden Knochenverankerungselemente 202, 202' mit einer Kraft F zusammengedrückt werden. Durch die Kraft F wirkt ein Biegemoment auf den Stab, das zu einem Drehmoment M auf die Knochenverankerungselemente 202, 202' um die Schraubenachse führt. Entsprechend führt Auseinanderziehen der Knochenverankerungselemente mit einer Kraft F zu einem Drehmoment M in entgegengesetzter Richtung um die schraubenachse.

Aus der US 2003/0045879 A1 ist ein Verankerungselement zum Verankern eines stabförmigen Elements in einem Knochen oder Wirbel bekannt, welches einen in dem Knochen oder Wirbel zu verankernden Schaft und ein mit dem Schaft verbundenes Aufnahmeteil zur Aufnahme des stabförmigen Elements und eine Fixationsvorrichtung zum Fixieren des stabförmigen Elements in dem Aufnahmeteil aufweist. Der Schaft ist über eine erste Komponente mit einem seitlichen Fortsatz mit dem Aufnahmeteil verbunden. Die erste Komponente hat eine seitliche Öffnung, in die der Kopf, der das Ende des Schafts bildet, einführbar ist.

Aus der US 2002/0143341 A1 ist ein Verankerungselement zum Verankern eines stabförmigen Elements in einem Knochen oder Wirbel bekannt mit einem in dem Knochen oder Wirbel zu verankerndem Schaft, einem mit dem Schaft direkt verbundenen Aufnahmeteil zur Aufnahme des stabförmigen Elements und einer Fixationsvorrichtung zum Fixieren des staubförmigen Elements in dem Aufnahmeteil. Der Schaft hat einen Kopf, der in dem Aufnahmeteil gehalten ist. Über ein speziell geformtes Druckelement und eine dazu geeignete Fixierungsvorrichtung kann der Stab unabhängig von dem Kopf fixiert werden.

Aus der US 6,368,321 B1 ist ein Verankerungselement zum Verankern eines stabförmigen Elements in einem Knochen oder Wirbel bekannt mit einem in dem Knochen oder Wirbel zu verankerndem Schaft, einem mit dem Schaft direkt verbundenen Aufnahmeteil zur Aufnahme des stabförmigen Elements und einer Fixationsvorrichtung zum Fixieren des stabförmigen Elements in dem Aufnahmeteil. Der Schaft hat einen Kopf, der in dem Aufnahmeteil gehalten ist. Über drei geeignete Fixierungsvorrichtungen kann der Stab unabhängig von dem Kopf fixiert werden.

Es ist Aufgabe der Erfindung, ein Verankerungselement und eine dynamischen Stabilisierungseinrichtung zur Stabilisierung und zur Bewegungsbegrenzung von benachbarten Wirbeln oder Knochen bereitzustellen, bei dem bzw. der ein Lockern oder Lösen des verankerungselementes im Betrieb verhindert wird.

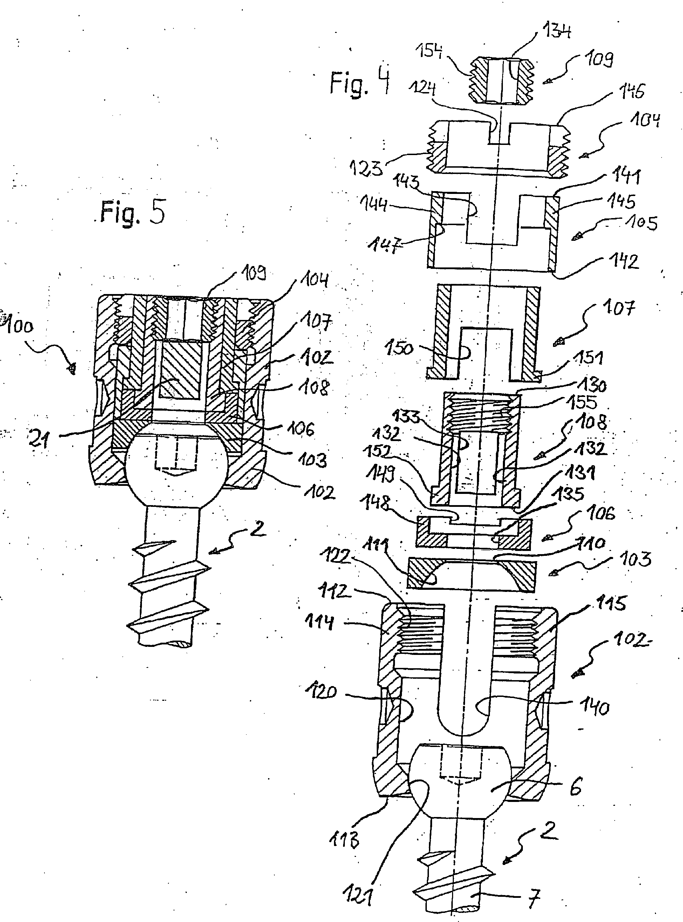

Die Aufgabe wird gelöst durch ein Verankerungselement nach Patentanspruch 1 und durch eine Stabilisierungsvorrichtung nach Patentanspruch 12. Die Erfindung ist in den Figuren 4 bis 8c dargestellt. Die restlichen Figuren veranschaulichen Beispiele, die nützlich für das Verständnis der Erfindung sind.

Weiterbildungen der Erfindung sind in den Unteransprüchen angegeben.

Die Erfindung weist den Vorteil auf, dass durch eine drehbare Verbindung des im Knochen verankerten Abschnitts des Verankerungselementes relativ zu dem Stab ein Lockern oder Lösen des Verankerungselementes durch an dem Verankerungselement angreifende Drehmomente effektiv verhindert werden kann. Die Stabilisierungseinrichtung ist vorteilhaft anwendbar zur Entkoppelung der Schaftrotation von Kopf- bzw. Stabfixierung bei der dynamischen Stabilisierung von Wirbeln.

Weitere Merkmale und Zweckmäßigkeiten der Erfindung ergeben sich aus der Beschreibung von Ausführungsbeispielen anhand der Figuren.

Von den Figuren zeigen:
- Fig. 1a: eine Explosionsdarstellung eines Verankerungelementes nach einer ersten Ausführungsform.
- Fig. 1b: eine teilgeschnittene Ansicht des Verankerungselements nach der ersten Ausfüh rungsform mit einem eingelegten Stab;
- FIG. 2: eine perspektivische Ansicht eines Lagerteils, das bei der ersten Ausführungsform verwendet wird;
- Fig. 3: Eine Abwandlung des Verankerungselementes nach der ersten Ausführungsform;
- Fig. 4: eine Explosionsdarstellung eines Verankerungselementes nach einer zweiten Ausführungsform der Erfindung;
- Fig. 5: eine teilgeschnittene Darstellung des Verankerungselementes nach der zweiten Ausführungsform der Erfindung;
- Fig. 6a: eine Seitenansicht der Stabfassung des Verankerungselements nach der zweiten Ausführungsform mit eingelegtem Stab und mit noch nicht vollständig eingeschraubter Innenschraube;
- Fig. 6b: eine Schnittdarstellung der Stabfassung des Verankerungselements nach der zweiten Ausführungsform mit eingelegtem Stab und mit vollständig eingeschraubter Innenschraube;
- Fig. 7a und 7b: ein Verankerungselement nach der zweiten Ausführungsform der Erfindung in zwei verschiedenen Winkelstellungen des stabförmigen Elements relativ zum Aufnahmeteil;
- Fig. 8a: eine Explosionsdarstellung eines Verankerungselements nach einer dritten Ausführungsform der Erfindung;
- Fig. 8b: Knochenschraube mit drehbarer Verbindung zwischen Kopf und Verankerungsabschnitt die bei der dritten Ausführungsform der Erfindung verwendet wird;
- Fig. 8c: Abwandlung des Verankerungselementes aus Fig. 8a und 8b; und
- Fig. 9: das Zustandekommen eines Drehmomentes auf eine Knochenschraube bei einer der Anmelderin bekannten dynamischen Stabilisierungsvorrichtung.;

### Erste Ausführungsform - nicht Teil der Erfindung

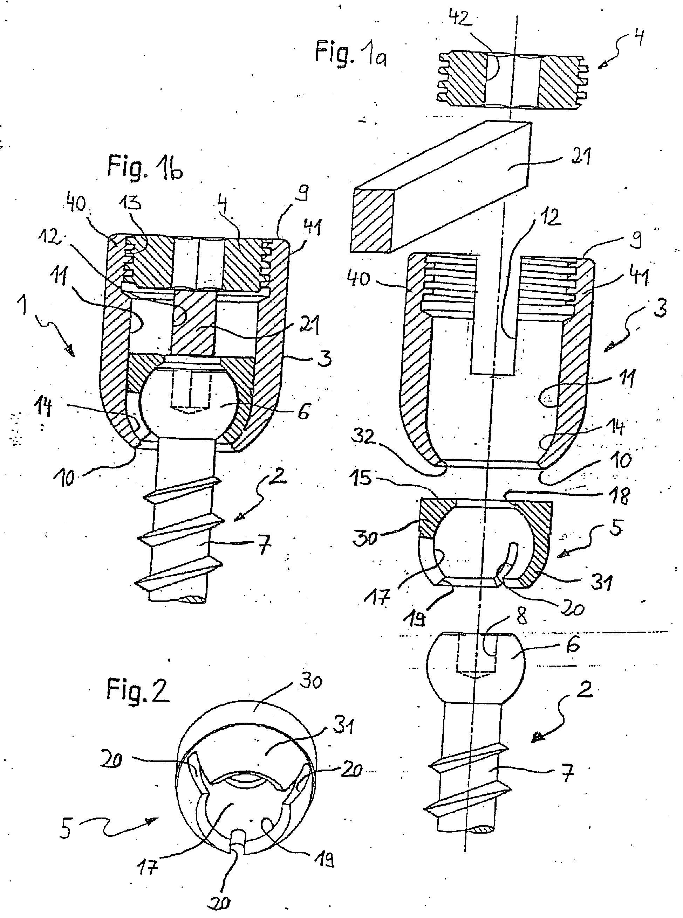

Wie in den Figuren 1a und 1b zu sehen ist, beinhaltet ein Verankerungselement 1 zum Verbinden eines Knochenteils oder eines Wirbels und eines Stabs 21 mit bevorzugt rechteckigem Querschnitt nach einer ersten Ausführungsform ein Schraubenelement 2, ein Aufnahmeteil 3, eine in das Aufnahmeteil einzuschraubenden Innenschraube 4 und ein Lagerteil 5.

Das Schraubenelement 2 weist einen Kopf 6 in der Form eines Kugelsegments und einen Gewindeschaft 7 zum Verankern in dem Knochen oder Wirbel auf. Auf der dem Gewindeschaft 7 abgewandten Seite ist der Kopf 6 des Schraubenelementes 2 abgeflacht und weist eine Ausnehmung 8 zum Ineingriffbringen eines Inbusschlüssels auf.

Das Aufnahmeteil 3 ist ein im wesentlichen zylinderförmiger Körper mit einem ersten Ende 9 und einem diesem gegenüberliegenden zweiten Ende 10. Koaxial zu seiner Hauptachse weist das Aufnahmeteil eine Bohrung 11 auf. Angrenzend an das erste Ende 9 ist zum Aufnehmen des Stabs 21 eine rechteckige Ausnehmung 12 vorgesehen, durch die zwei freie Schenkel 40, 41 gebildet sind. Die Breite der Ausnehmung ist dabei etwas größer als die Länge der schmalen Seite des Stabs, während die Tiefe der Ausnehmung größer als die Länge der breiten Seite des Stabs ist. Auf der Innenseite der Schenkel 40, 41 ist angrenzend an das erste Ende 9 in der Bohrung 11 ein Innengewinde 13 vorgesehen. In einem an das erste Ende angrenzenden ersten Abschnitt weist die Bohrung einen im wesentlichen konstanten Innendurchmesser auf, der größer als der Durchmesser des Kopfes 6 des Schraubenelementes 2 ist. An den ersten Abschnitt angrenzend weist das Aufnahmeteil 3 einen sich in Richtung von dem ersten Ende 9 zu dem zweiten Ende 10 hin derart verjüngenden Abschnitt auf, dass ein an das zweite Ende angrenzender sphärischer Sitz 14 ausgebildet ist. Die Öffnung 32 auf der Seite des zweiten Endes ist größer als der Durchmesser des Gewindeschaftes 7 des Schraubenelementes 2.

Das Lagerteil 5 weist einen zylindrischen Abschnitt mit flacher Stirnseite 15 auf. Der Durchmesser des zylindrischen Abschnittes ist so gewählt, dass im zusammengebauten Zustand dieser Abschnitt mit Presspassung in dem ersten Abschnitt des Aufnahmeteils 3 sitzt. Das Lagerteil 5 weist weiter einen an den zylindrischen Abschnitt angrenzenden kugelschalenförmigen Abschnitt 31 auf, dessen äußere Form an die Form des sphärischen Sitzes 14 angepasst ist. Im Inneren des Lagerteils 3 ist eine sphärische Ausnehmung 17 vorgesehen, die der Aufnahme des Kopfes 6 des Schraubenelementes 2 dient und an dessen sphärische Form angepasst ist. Der Innendurchmesser der Ausnehmung 17 ist, je nach gewünschter Schwergängigkeit der Dreh- und Schwenkbewegung des Schraubenelementes relativ zu dem Lagerteil, in einem Bereich von etwa gleich oder gerade etwas größer als der Durchmesser des Kopfes des Schraubenelementes. Von der flachen Stirnseite 15 ist eine in die Ausnehmung 17 einmündende Bohrung 18 mit einem Durchmesser vorgesehen, der das Durchführen eines Inbusschlüssels zum Festschrauben des Schraubenelementes 2 erlaubt, aber geringer ist als der Durchmesser des Kopfes 6 des Schraubenelementes 2. In dem kugelschalenförmigen Abschnitt ist eine in die Ausnehmung 17 einmündende koaxiale Bohrung 19 vorgesehen, deren Durchmesser kleiner als der Durchmesser des Kopfes 6 des Schraubenelementes 2, aber größer als der Durchmesser des Gewindeabschnittes 7 ist. Wie in Fig. 2 zu sehen weist das Lagerteil 5 in dem kugelschalenförmigen Abschnitt angrenzend an die der flachen Stirnseite gegenüberliegende Seite des Lagerteils Schlitze 20 auf, durch die die Elastizität des Lagerteils erhöht ist. Vorzugsweise ist das Lagerteil 5 aus einem körperverträglichen Kunststoff gefertigt, der gute Gleiteigenschaften aufweist. Bevorzugt wird Polyethylen (PE) eingesetzt. Hierbei können verschiedene Vernetzungsgrade mit daraus resultierenden verschiedenen Molekulargewichten, wie z.B. LDPE und LLDPE mit Molekulargewichten von bis zu 50.000 g/mol, HDPE mit Molekulargewichten bis zu 200.000 g/mol oder UHMWP (Ultra-Height Molecular Weight Poly Ethylen) mit Molekulargewichten um 6.000.000 g/mol, verwendet werden. Bevorzugt wird für das Lagerteil aufgrund seines geringen Langzeitverschleißes UHMWP eingesetzt. Die Innenschraube 4 weist eine koaxiale Ausnehmung 42 zum Ineingriffbringen eines Inbusschlüssels auf.

Im Betrieb wird zunächst der Kopf 6 des Schraubenelementes 2 in die Ausnehmung 17 des Lagerteils 5 eingebracht und dann das Schraubenelement 2 zusammen mit dem Lagerteil 5 in das Aufnahmeteil 3 eingeführt. Anschließend an diese Vormontage wird das Schraubenelement 2 in den Knochen bzw. Wirbel eingeschraubt. Danach wird das stabförmige Element 21 in das Aufnahmeteil 3 eingelegt, wodurch sich das Aufnahmeteil 3 selbst richtig zum stabförmigen Element 21 hin ausrichtet. Anschließend wird das stabförmige Element 21 durch die Innenschraube 4 relativ zu dem Aufnahmeteil 3 fixiert.

Auf die oben beschriebene Art und Weise wird eine Verbindung zwischen dem fest in den Knochen bzw. Wirbel eingeschraubten Schraubenelement 2 und dem stabförmigen Element 21 hergestellt, bei der der Kopf 6 des Schraubenelementes 2 in dem Lagerteil 5 in einem vorbestimmten Raumwinkelbereich drehbar gelagert ist. Dabei ist der Raumwinkelbereich einerseits durch den Durchmesser des Gewindeschaftes 7 und andererseits durch den Durchmesser der Öffnung 32 auf der Seite des zweiten Endes 10 des Aufnahmeteils 3 oder den Durchmesser der koaxialen Bohrung 19 in dem Aufnahmeteil 5 festgelegt. Je nachdem, wie der Durchmesser der sphärischen Ausnehmung 17 und der Durchmesser des Kopfes 6 zueinander gewählt werden, ist die Einstellung unterschiedlich starker Reibungskräfte zwischen Kopf und Lagerteil und somit die Einstellung der Kräfte möglich, die überwunden werden müssen, um den Kopf 6 des Schraubenelements 2 in der Ausnehmung 17 des Lagerteils 3 zu drehen bzw. zu schwenken.

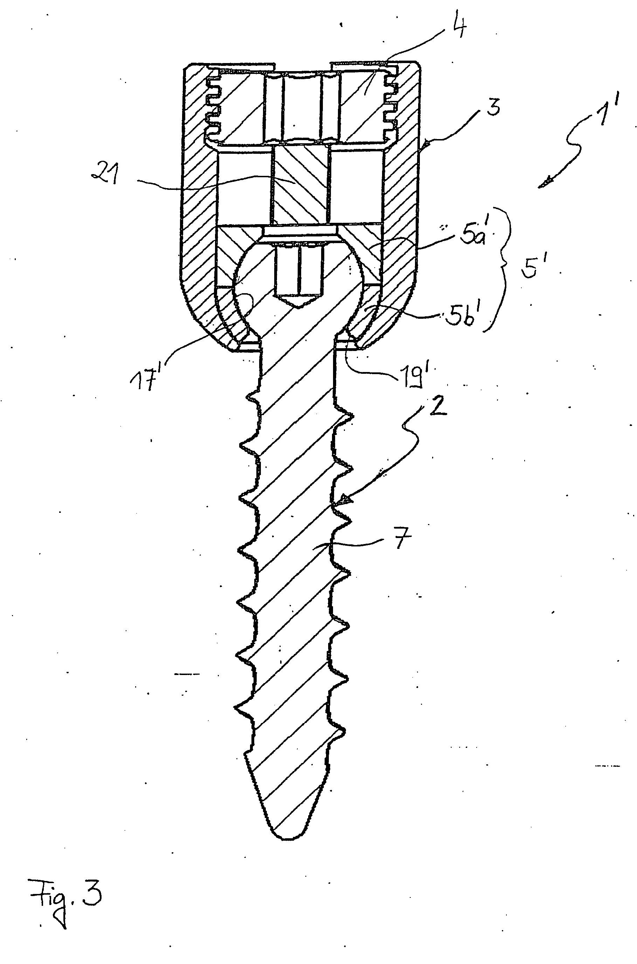

In Fig. 3 ist eine Abwandlung 1' des Verankerungselementes 1 nach der ersten Ausführungsform dargestellt, bei der das Lagerteil 5' zweiteilig aus einem ersten Lagerelement 5a' und einem zweiten Lagerelement 5b' ausgebildet ist. Das zweiteilige Lagerteil 5' ist wie das Lagerteil 5, jedoch parallel zur Stirnseite in zwei Teile geschnitten ausgebildet. Durch die zweiteilige Form kann der Kopf 6 des Schraubenelementes 2 in die Ausnehmung 17' eingebracht werden, ohne die Öffnung 19' aufzuspreizen. Das Lagerteil 5' kann daher aus einem steifen Material ohne Schlitze ausgebildet sein.

### Zweite Ausführungsform

In den Figuren 4 bis 7 ist ein Verankerungselement 100 nach einer zweiten Ausführungsform der Erfindung dargestellt. Elemente, die gleich denjenigen der ersten Ausführungsform sind, sind mit gleichen Bezugszeichen versehen.

Wie am besten in den Figuren 4 und 5 zu sehen ist, beinhaltet das Verankerungselement 100 nach der zweiten Ausführungsform ein Schraubenelement 2, ein Aufnahmeteil 102, ein Druckelement 103, einen ersten Ring 104, einen zweiten Ring 105, ein erstes Lagerteil 106, ein zweites Lagerteil 107, eine Stabfassung 108 und eine Innenschraube 109.

Das Schraubenelement 2 in dem Verankerungselement 100 nach der zweiten Ausführungsform ist identisch mit dem Schraubenelement 2 des Verankerungselements 1 nach der ersten Ausführungsform.

Das Aufnahmeteil 102 ist ein im wesentlichen zylindrischer Körper mit einem ersten Ende 112 und einem diesem gegenüberliegenden zweiten Ende 113. Eine koaxiale Bohrung 120 erstreckt sich von dem ersten Ende 112 bis zu dem zweiten Ende 113 des Aufnahmeteils 102. Angrenzend an das erste Ende 112 ist eine im wesentlichen U-förmige Ausnehmung 140 vorgesehen, wodurch zwei freie Schenkel 114 und 115 gebildet sind. Angrenzend an das erste Ende 112 ist auf der Innenseite der freien Schenkel 114 und 115 ein Innengewinde 122 vorgesehen. In einem an das erste Ende 112 des Aufnahmeteils 102 angrenzenden Abschnitt hat die Bohrung 120 einen im wesentlichen konstanten Durchmesser, der größer ist, als der Durchmesser des Kopfes 6 des Schraubenelementes 2. In einem sich an den ersten Abschnitt anschließenden und bis zum zweiten Ende des Aufnahmeteils 102 erstreckenden zweiten Abschnitt verjüngt sich die Bohrung 120 in Richtung des zweiten Endes 113. Angrenzend an das zweite Ende 113 ist ein sphärischer Abschnitt 121 gebildet, dessen Form an die Form des Kopfes 6 des Schraubenelementes 2 angepasst ist. Der Durchmesser der Bohrung in dem zweiten Abschnitt ist so gewählt, dass er angrenzend an das zweite Ende kleiner als der Durchmesser des Kopfes 6 des Schraubenelementes 2, aber größer als der Durchmesser des Gewindeschaftes 7 des Schraubenelementes 2 ist.

Der erste Ring 104 weist auf seiner Außenseite ein Außengewinde 123 auf, das mit dem Innengewinde 122 auf der Innenseite der freien Schenkel 114 und 115 des Aufnahmeteils 102 zusammenwirkt. An einer Stirnseite 146 des ersten Rings 104 sind sich in radialer Richtung erstreckende Ausnehmungen 124 vorgesehen, in die ein Werkzeug zum Einschrauben des ersten Rings 104 in das Aufnahmeteil 102 eingreifen kann.

Der zweite Ring 105 ist rohrförmig ausgebildet mit konstantem Außendurchmesser mit einem an ein erstes Ende 141 angrenzenden ersten Abschnitt mit einem ersten Innendurchmesser und mit einem an ein zweites Ende 142 angrenzenden zweiten Abschnitt mit einem zweiten Innendurchmesser, der größer ist als der Innendurchmesser des ersten Abschnitts, so dass dadurch eine Schulter 147 ausgebildet ist. Der Außendurchmesser des zweiten Ringes 105 ist über die gesamte Länge des Ringes konstant und etwas kleiner als der Durchmesser der Bohrung 120 in dem an das erste Ende 112 des Aufnahmeteils 102 angrenzenden Abschnitt, sodass der Ring 105 in die Bohrung 120 einschiebbar ist. An das erste Ende 141 angrenzend ist eine rechteckige Ausnehmung 143 vorgesehen, durch die zwei freie Schenkel 144, 145 gebildet sind. Die Breite der Ausnehmung 143 ist wie die Breite der U-förmigen Ausnehmung 140 des Aufnahmeteils 102 größer als die schmale Seite des rechteckigen Querschnittes des Stabes, sodass der in diese Ausnehmungen eingelegte Stab 21 in einem vorbestimmten Winkelbereich, bevorzugt etwa ±10°, hin und her geschwenkt werden kann.

Das Druckelement 103 hat im wesentlichen die Form eines flachen Zylinders mit einer sphärischen Ausnehmung 111 auf der dem Schraubenkopf zugewandten Seite, deren Form an die Form des Kopfes 6 des Schraubenelementes 2 angepasst ist, und mit einer in diese Ausnehmung 111 einmündenden koaxialen Bohrung 110 zum Hindurchführen eines Schraubendrehers versehen. Der Außendurchmesser des Druckelementes 103 ist etwas geringer als der Durchmesser der Bohrung 120 in dem Aufnahmeteil 102, sodass das Druckelement 103 in die Bohrung des Aufnahmeteils einschiebbar ist.

Der erste und der zweite Ring 104, 105 dienen zum Ausüben von Druck auf das Druckelement 103 und damit zum Fixieren des Kopfes 6 des Schraubenelementes 2 in dem sphärischen Abschnitt 121.

Das erste Lagerteil 106 hat die Form einer Kreisscheibe mit einer koaxialen Bohrung 135 zum Hindurchführen eines Schraubendrehers und mit einem in Umfangsrichtung verlaufenden ringförmigen Vorsprung 148, der im eingesetzten Zustand auf der dem Druckelement abgewandten Seite liegt. Der ringförmige Vorsprung weist zwei gegenüberliegende rechteckige Ausnehmungen 149 auf.

Das zweite Lagerteil 107 ist als ein rohrförmiger Abschnitt mit einem flanschartigen Ansatz 151 ausgebildet, wobei der Durchmesser des rohrförmigen Abschnittes geringer ist als der Durchmesser des ersten Lagerteils 106. Der äußere Durchmesser des flanschartigen Ansatzes 151 des zweiten Lagerteils 107 ist gleich dem Durchmesser des ersten Lagerteils 106. Angrenzend an die Seite mit dem flanschartigen Ansatz 151 sind zwei sich gegenüberliegende rechteckige Ausnehmungen 150 vorgesehen.

Das erste und das zweite Lagerteil 106, 107 sind im eingesetzten Zustand koaxial so angeordnet, dass der ringförmige Vorsprung 148 des ersten Lagerteils 106 an den flanschartigen Ansatz 151 des zweiten Lagerteils 107 angrenzt, wobei die rechteckigen Ausnehmungen 149, 150 in den beiden Lagerteilen 106, 107 jeweils aneinander ausgerichtet sind, sodass in dem durch die beiden Lagerteile 106, 107 gebildeten Lager zwei sich gegenüberliegende, in das Innere des Lagers mündende Öffnungen zum Aufnehmen des Stabes ausgebildet sind. In der Breite sind die durch die Ausnehmungen 149, 150 im zusammengesetzten Zustand ausgebildeten Öffnungen so ausgelegt, dass der durch diese Öffnungen eingelegte Stab 21 in einem vorbestimmten Winkelbereich, bevorzugt etwa ±10°, hin und her geschwenkt werden kann. Die Höhe der durch die Ausnehmungen 149, 150 im zusammengesetzten Zustand gebildeten Öffnungen ist etwas größer als die entsprechende Querschnittsseite des Stabs 21. Der Außendurchmesser des Lagers ist gerade so bemessen, dass eine Presspassung des Lagers in dem ersten und zweiten Klemmring möglich ist. Im zusammengebauten Zustand liegt der flanschartige Ansatz 151 an der Schulter 147 des zweiten Rings 105 an.

Das erste und das zweite Lagerteil 106, 107 sind vorzugsweise aus einem körperverträglichen Kunststoff gefertigt, der gute Gleiteigenschaften aufweist. Bevorzugt wird Polyethylen (PE) eingesetzt. Hierbei können verschiedene Vernetzungsgrade mit daraus resultierenden verschiedenen Molekulargewichten, wie z.B. LDPE und LLDPE mit Molekulargewichten von bis zu 50.000 g/mol, HDPE mit Molekulargewichten bis zu 200.000 g/mol oder UHMWP (Ultra-Height Molecular Weight Poly Ethylen) mit Molekulargewichten um 6.000.000 g/mol, verwendet werden. Bevorzugt wird für das Lagerteil aufgrund seines geringen Langzeitverschleißes UHMWP eingesetzt. Die übrigen Teile des Verankerungselementes sind bevorzugt aus einem körperverträglichen Material mit guten mechanischen Eigenschaften, wie z.B. Titan, gefertigt.

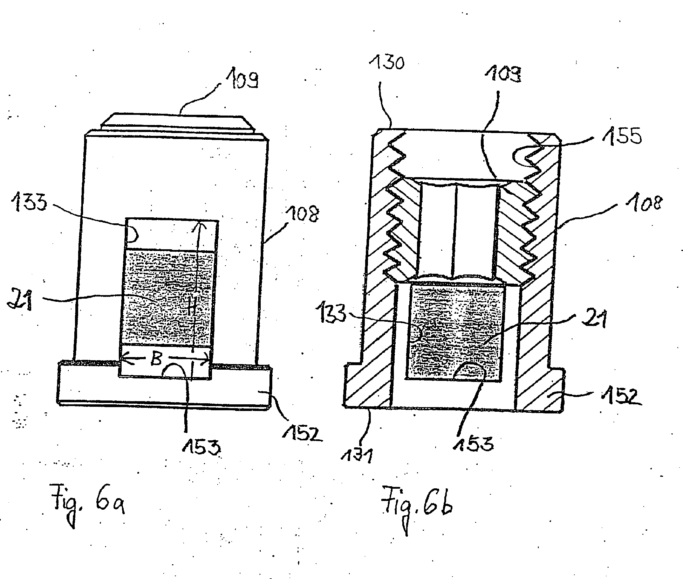

Wie aus den Figuren 5, 6a und 6b ersichtlich ist, ist die Stabfassung 108 als zylinderförmiger Körper mit einem ersten Ende 130 und einem zweiten Ende 131 ausgebildet. Von dem ersten Ende 130 ist bis zu dem zweiten Ende 131 eine durchgehende koaxiale Bohrung 132 vorgesehen. Angrenzend an das erste Ende 130 ist in der Bohrung 132 ein Innengewinde 155 vorgesehen, in das die Innenschraube 109 eingeschraubt werden kann. Der Außendurchmesser der Stabfassung 108 ist etwas geringer als der Innendurchmesser des zweiten Lagerteils 107. An seinem zweiten Ende 131 weist die Stabfassung 108 einen flanschartigen Ansatz 152 auf, dessen Außendurchmesser etwas geringer als der Innendurchmesser des ersten Lagerteils 106 ist. An den Seitenwänden der Stabfassung 108 sind zwei sich gegenüberliegende Öffnungen 133 mit rechteckigem Querschnitt vorgesehen. Die Breite B der Öffnung ist etwas größer als die Breite des Stabes. Die Höhe H der Öffnung ist größer als die Höhe des Stabes.

Die Innenschraube 109 weist ein Außengewinde 154 auf, das mit dem Innengewinde 155 der Stabfassung 108 zusammenwirkt. Eine koaxiale Bohrung 134 durch die Innenschraube 109 weist einen Querschnitt auf, der zum Ineingriffbringen mit einem Inbusschlüssel geeignet ist.

Wie aus den Figuren 6a und 6b ersichtlich ist, ist die axiale Länge des Innengewindeabschnitts 155 der Stabfassung 108 und die Höhe der Öffnungen 133 so gewählt, dass der Stab durch Festziehen der Innenschraube 109 von einer Position, in der er in der Öffnung verschiebbar ist, in eine Position, in der er gegen den unteren Rand 153 der Öffnung 133 gedrückt und so fixiert wird, verschiebbar ist.

Im Betrieb wird zum Vormontieren des Verankerungselements zuerst das Schraubenelement 2 mit dem Gewindeschaft 7 voran in das Aufnahmeteil 102 eingeführt, sodass der Kopf 6 an der sphärischen Fläche 121 anliegt. Im Anschluss daran wird von dem ersten Ende 112 des Aufnahmeteils 102 her in die koaxiale Bohrung 120 des Aufnahmeteils 102 zuerst das Druckelement 103 mit der sphärischen Ausnehmung 111 gegen den Kopf hin eingeführt, dann wird das erste Lagerteil 106 mit der koaxialen Bohrung voran in das Aufnahmeteil 102 eingeführt und anschließend wird die Stabfassung 108 mit zuvor leicht eingeschraubter Innenschraube 109 in das erste Lagerteil 106 eingelegt. Hierauf wird nacheinander das zweite Lagerteil 107 und der zweite Ring 105 zwischen Seitenwand des Aufnahmeteils 102 und das erste und zweite Lagerteil 106, 107 eingebracht. Schließlich wird der erste Ring 104 in das Aufnahmeteil 102 gerade soweit eingeschraubt, dass ein Herausfallen der in das Aufnahmeteil 102 eingebrachten Elemente verhindert wird. Hiermit ist die Vormontage des Verankerungselementes 100 abgeschlossen.

Alternativ dazu können aber auch die Stabfassung 108, das erste und das zweite Lagerteil 106, 107, der erste und der zweite Ring 104, 105 und die Innenschraube 109 auch zuerst außerhalb des Aufnahmeteils zusammengesetzt und erst dann in das Aufnahmeteil eingeführt werden. Weitere Arten des Zusammenbaus sind möglich.

Im Betrieb bei der Operation wird mit einem Inbusschlüssel durch die Bohrungen 134, 132, 135 und 110 hindurch das Schraubenelement 2 zuerst in den Wirbel bzw. Knochen eingeschraubt. Danach wird von der Seite des Aufnahmeteils 102 her der Stab zwischen den beiden freien Schenkeln 114 und 115 des Aufnahmeteils 102 durch die Öffnungen 133 in der Stabfassung 108 und durch die Öffnungen in dem ersten und zweiten Lagerteil 106 und 107, sowie durch die Ausnehmungen in dem ersten Ring 105 eingeschoben. Anschließend wird durch Festziehen des ersten Rings 104 auf das Druckelement 103 eine Kraft ausgeübt und so das Aufnahmeteil 102 relativ zu dem Schraubenelement 2 fixiert. Dann wird durch Einsetzen und Festziehen der Innenschraube 109 der Stab 21 in der Stabfassung 108 fixiert.

So entsteht eine Verbindung zwischen dem stabförmigen Element 21 und dem Knochen bzw. Wirbel, bei der sich die Stabfassung 108 mit dem fixierten stabförmigen Element 21 um die Hauptachse des Aufnahmeteils 102 in einem vorbestimmten Winkelbereich drehen kann. Der Winkelbereich ist dabei durch die Abmessung des Stabes 21 und die Breite der Ausnehmung 140 in dem Aufnahmeteil 102, die Breite der rechteckigen Ausnehmungen 149, 150 in dem ersten bzw. dem zweiten Lagerteil 106, 107 der Öffnungen 149 und die Breite der Ausnehmungen 143 in dem zweiten Ring 105 festgelegt. Die Stabfassung 108 dreht sich mit dem Stab 21 mit, während das Lagerteil 106, 107 durch Presspassung fest in dem ersten und zweiten Ring 104, 105 sitzt. Die Winkelstellung der Schraubenachse relativ zu dem Aufnahmeteil 102 bleibt erhalten.

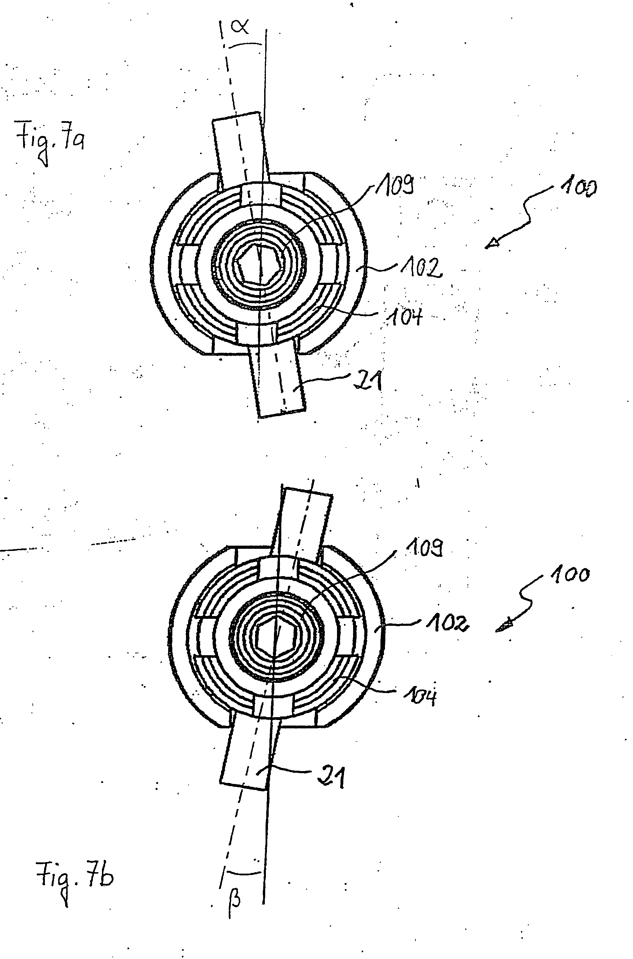

In den Figuren 7a und 7b sind zwei verschiedene Grenzwinkelstellungen α, β des stabförmigen Elementes 21 gegenüber dem Aufnahmeteil 102 dargestellt.

Im Gegensatz zum Verankerungselement 1 nach der ersten Ausführungsform, das drei Rotationsfreiheitsgrade des Stabes relativ zum Schraubenelement aufweist, weist die Verbindung mit einem Verankerungselement 100 nach der zweiten Ausführungsform nur einen Rotationsfreiheitsgrad auf.

### Dritte Ausführungsform

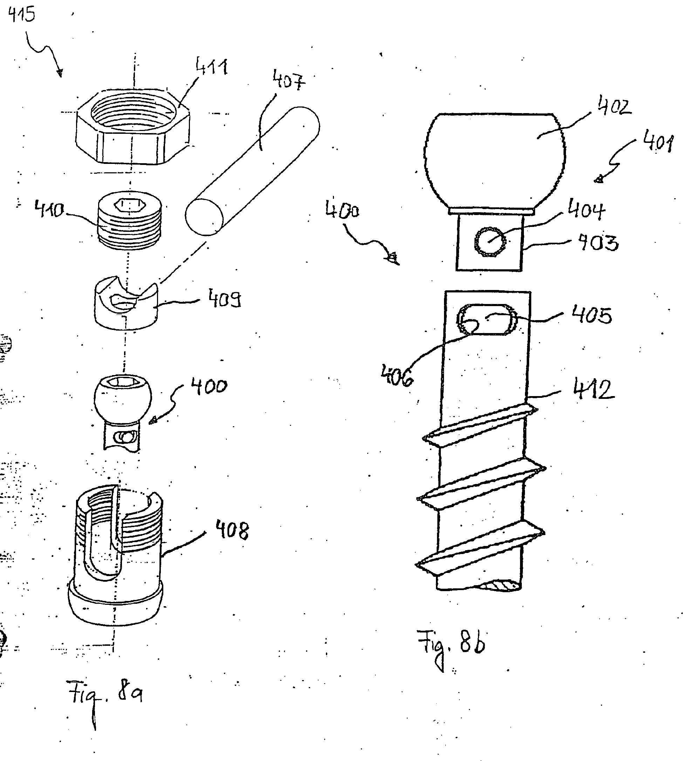

Nach einer dritten Ausführungsform der Erfindung wird eine drehbare Verbindung zwischen einem Stab und einem Knochen bzw. Wirbel dadurch hergestellt, indem wie in Fig. 8a dargestellt in einer Polyaxialschraube, bei der der Winkel zwischen Stab 407 und Aufnahmeteil 408, sowie zwischen Schraubenelement und Aufnahmeteil fixiert ist, ein zweiteiliges Schraubenelement 400 verwendet wird, bei dem der Kopf 401 des Schraubenelementes drehbar mit dem Gewindeschaft 412 verbunden ist. Die Polyaxialschraube beinhaltet wie in Fig. 8a dargestellt ein Aufnahmeteil 408, ein Druckelement 409, eine Innenschraube 410 und eine Außenschraube 411.

Wie aus Fig. 8b ersichtlich ist besteht der Kopf 401 des Schraubenelementes 400 aus einem kugelsegmentförmigen Kopfabschnitt 402 mit einem zylindrischen Hals 403. An der Seitenfläche des Halses 403 ist weiter ein Stift 404 vorgesehen, der entlang seiner Längsachse gegen eine Federkraft in den Hals 403 eindrückbar ist.

Der Gewindeschaft 412 weist an seiner dem Kopf 401 des Schraubenelementes 400 zugewandten Seite eine koaxiale Ausnehmung 405 auf, in welche der Hals 403 eingreift. In der Seitenwand dieser Ausnehmung 405 ist ein Langloch 406 vorgesehen, in welches der Stift 404 eingreift.

Im Betrieb wird der Stift in den Hals soweit eingedrückt, dass der Hals 403 in die Ausnehmung 405 des Gewindeschaftes 412 eingeschoben werden kann. Der Hals 403 wird dann derart in die Ausnehmung 405 eingeschoben, dass der Stift 404 von der Federkraft nach außen gedrückt in das Langloch 406 in der Wand der Ausnehmung 405 eingreift. Dadurch wird eine Verbindung zwischen dem Kopf 401 und dem Gewindeschaft 412 des Schraubenelementes 400 gebildet, bei der der Kopf 401 gegen den Gewindeschaft 412 des Schraubenelementes innerhalb eines durch die Länge des Langlochs 406 vorbestimmten Winkelbereichs koaxial gegeneinander gedreht werden können.

Das Schraubenelement 400 wird dann in ein Aufnahmeteil 408 eingeführt und in den Knochen eingeschraubt. Anschließend wird in bekannter Weise die Stellung des Schraubenelements zum Aufnahmeteil fixiert, sowie der Stab 407 eingelegt und fixiert.

Die Verbindung mit einem Verankerungselement nach der dritten Ausführungsform weist wie bei der zweiten Ausführungsform einen Rotationsfreiheitsgrad auf.

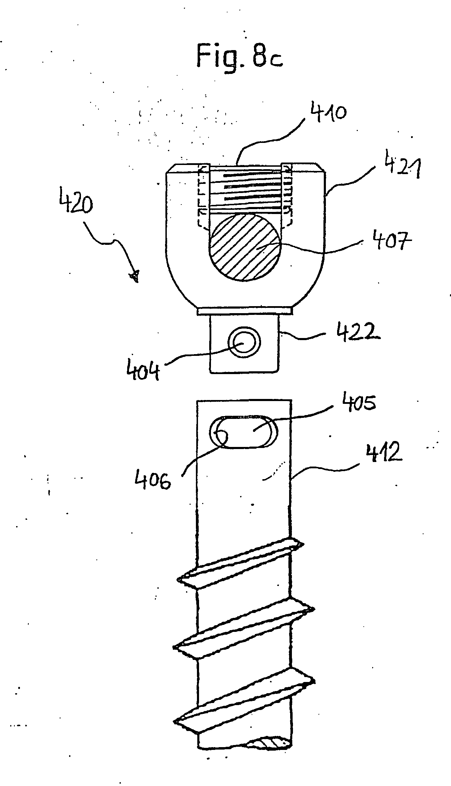

In einer in Fig. 8c dargestellten Abwandlung der dritten Ausführungsform ist das Knochenverankerungselement 420 als Monoaxialschraube ausgebildet, bei der das Aufnahmeteil 421 fest mit dem Kopf des zweiteiligen Schraubenelementes verbunden ist bzw. ein integraler Bestandteil desselben ist. Im übrigen ist das Knochenverankerungselement 420 wie die zuvor beschriebene dritte Ausführungsform ausgebildet.

Abwandlungen der zuvor beschriebenen Ausführungsformen sind möglich.

Die Öffnung 19 des Verankerungselementes nach der ersten Ausführungsform wurde so beschrieben, dass ihr Durchmesser kleiner als der des Kopfes 6, aber größer als der Durchmesser des Gewindeabschnittes 7 ist. Der Durchmesser der Öffnung 19 kann aber auch kleiner als der Durchmesser des Gewindeschaftes 7 sein, wenn das Schraubenelement 2 zweiteilig ausgebildet ist, sodass der Gewindeschaft beim Zusammenbau nicht durch die Öffnung 19 hindurchgeführt werden muss. Weiter ist es möglich, dass die Öffnung so ausgebildet ist, dass das Schraubenelement durch diese hindurchgeschraubt werden kann.

Bei dem Verankerungselement nach der ersten Ausführungsform kann eine nicht-sphärische aber bezüglich der Schraubenachse rotationssymmetrische Form des Kopfes eine Einschränkung der Drehbewegung des Schraubenelementes relativ zu dem Aufnahmeteil auf einen Freiheitsgrad bewirken. Das Lagerteil 5 nach der ersten Ausführungsform muss nicht zwingend wenigstens einen oder mehrere Schlitze 20 aufweisen, wenn die Elastizität des verwendeten Materials ein Einbringen des Kopfes 6 des Schraubenelementes 2 auch ohne Schlitze 20 erlaubt.

Der Durchmesser der Bohrung 120 in dem zweiten Abschnitt des Aufnahmeteils 102 nach der zweiten Ausführungsform ist so beschrieben worden, dass er angrenzend an das zweite Ende 113 größer als der Durchmesser des Gewindeschaftes 7 des Schraubenelementes 2 ist. Der Durchmesser der Bohrung 120 kann aber angrenzend an das zweite Ende auch so bemessen sein, dass das Schraubenelement durch die Bohrung hindurchgeschraubt wird oder aber, bei einem mehrteiligen Schraubenelement, geringer ist als der Durchmesser des Gewindeschafts 7, der in diesem Fall nicht durch die Bohrung 120 hindurchgeführt werden muss, sondern von außen mit dem in dem Aufnahmeteil befindlichen Kopf verbunden wird.

Weiter kann das erste Lagerteil 106 auch ohne den Vorsprung 148 ausgebildet sein.

Das Verankerungselement 100 nach der zweiten Ausführungsform kann als Monoaxialschraube ausgebildet sein, bei der das Aufnahmeteil 102 fest mit dem Schraubenelement 2 verbunden ist bzw. ein integraler Bestandteil desselben ist.

Bei allen beschriebenen Verankerungselementen kann anstelle der Verankerung mit einem Schraubenelement eine andere Art der Verankerung in dem Knochen bzw. Wirbel vorgesehen sein, wie z.B. die Verankerung durch Haken.

Die Knochenverankerungselemente nach der ersten und der zweiten Ausführungsform der Erfindung wurden für stabförmige Elemente mit quadratischen Querschnitt beschrieben. Durch entsprechende Abwandlung der Ausnehmungen und Bohrungen zur Aufnahme des Stabes können dies Knochenverankerungselementes auch für die Verwendung von stabförmigen Elementen mit einem kreisförmigen oder einem anderen Querschnitt angepasst werden. Ebenso kann auch das Knochenverankerungselement nach der dritten Ausführungsform für die Verwendung mit einem stabförmigen Element mit einem rechteckigen oder mit einem anderen Querschnitt abgewandelt werden.

Bei der dritten Ausführungsform der Erdindung wurde beschrieben, dass der Stift 404 gegen eine Federkraft in den Hals 403 eingedrückt werden kann. Der Stift 404 kann jedoch auch mit Presspassung in einem Loch in dem Hals 403 bzw. 422 eingebracht und so fest mit dem Hals 403 bzw. 422 verbunden sein. In diesem Fall ist im Betrieb der Hals zuerst ohne Stift in die Ausnehmung 405 einzuführen und dann der Stift durch das Langloch 406 hindurch in das Loch in dem Hals einzubringen. Es ist auch möglich, den Stift und den Hals so auszubilden, dass der Stift mit einem Außengewinde in ein in dem Loch des Halses vorgesehenes Innengewinde eingeschraubt werden kann. Eine Vielzahl weiterer Drehverbindungen zwischen Schraubenkopf 401 und dem Gewindeabschnitt 402 ist möglich.

Eine erfindungsgemäße Stabilisierungseinrichtung zur dynamischen Stabilisierung von Knochen bzw. Wirbeln besteht wie die in Fig. 9 dargestellte bekannte Stabilisierungseinrichtung aus wenigstens zwei mit einem stabförmigen Element verbundenen Knochenverankerungselementen von denen wenigstens eines ein Knochenverankerungselement nach der ersten bis dritten Ausführungsform ist.

Ein besonderer Aspekt sieht ein Verankerungselement (1, 1', 100, 415, 420) zum Verankern eines stabförmigen Elements (21, 407) in einem Knochen oder Wirbel vor, der einen in dem Knochen oder Wirbel zu verankernden Schaft (7, 412), einen mit dem Schaft (7, 412) verbundenen Aufnahmeteil (3, 102, 408, 421) zur Aufnahme des stabförmigen Elements (21, 407), und eine Fixationsvorrichtung (4, 109, 410) zum Fixieren des stabförmigen Elements (21, 407) in dem Aufnahmeteil (3, 102, 408, 421) besitzt. Kennzeichnend ist dabei, daß in fixiertem Zustand des stabförmigen Elements (21, 407) der Schaft (7, 412) über das Aufnahmeteil (3, 102, 408, 421) mit dem stabförmigen Element (21, 407) derart beweglich verbunden ist, daß der Schaft (7, 412) relativ zu dem stabförmigen Element (21, 407) eine Bewegung mit wenigstens einem Rotationsfreiheitsgrad, aber keinem Translationsfreiheitsgrad ausführen kann. Die im Hauptanspruch und in den Unteransprüchen angegebenen Weiterbildungen sind auch mit dieser Ausgestaltung kombinierbar.

## Patentansprüche

1. Verankerungselement (100, 415, 420) zum Verankern eines stabförmigen Elements (21, 407) in einem Knochen oder Wirbel
mit einem in dem Knochen oder Wirbel zu verankernden Schaft (7, 412),
einem mit dem Schaft (7, 412) verbundenen Aufnahmeteil (102, 408, 421) zur Aufnahme des stabförmigen Elements (21, 407), und
einer Fixationsvorrichtung (109, 410) zum Fixieren des stabförmigen Elements (21, 407) in dem Aufnahmeteil ( 102, 408, 421),
**dadurch gekennzeichnet, daß** in fixiertem Zustand des stabförmigen Elements (21, 407) der Schaft (7, 412) über das Aufnahmeteil (102, 408, 421) mit dem stabförmigen Element (21, 407) drehbar verbunden ist, und
eine Führungsfläche (152, 406) vorgesehen ist, welche die relative Bewegung zwischen dem Schaft (7, 412) und dem stabförmigen Element (21, 407) auf einen Rotationsfreiheitsgrad einschränkt.

2. Verankerungselement (100) nach Anspruch 1, wobei der Schaft (7) mit dem Aufnahmeteil (102) fest verbunden ist und das stabförmige Element (21) in einer Fassung (108) in dem Aufnahmeteil (102) drehbar gehalten ist.

3. Verankerungselement (100) nach Anspruch 2,
wobei die Fassung (108) in einem Lagerelement (106, 107) drehbar gelagert ist.

4. Verankerungselement (100) nach Anspruch 3, das als Polyaxialschraube mit einem aus dem Schaft (7) und einem kugelsegmentförmigen Kopf (6) bestehenden Knochenverankerungselement (2) ausgebildet ist, wobei
an das eine Ende des Aufnahmeteils (102) angrenzend ein sphärischer Sitz (121) zur Aufnahme des kugelsegmentförmigen Kopfes (6) des Knochenverankerungselementes (2) vorgesehen ist,
an das andere Ende des Aufnahmeteils (102) angrenzend zwei sich gegenüberliegende Ausnehmungen (140) vorgesehen sind, durch die zwei freie Schenkel (114, 115) gebildet werden,
an den Innenseiten der freien Schenkel (114, 115) ein Gewinde (122) vorgesehen ist, in welches ein ringförmiges Element (104) einschraubbar ist, mit dem direkt oder indirekt Druck auf ein Druckelement (103) ausgeübt wird, welches diesen Druck wiederum an den Kopf (6) des Knochenverankerungselementes (2) weitergibt, sodass das Knochenverankerungselement (2) in einer vorbestimmten Winkelstellung relativ zu dem Aufnahmeteil (102) fixiert wird, wobei
das Lagerelement (106, 107) innerhalb des ringförmigen Elementes (104) sitzt.

5. Verankerungselement (100) nach Anspruch 4, wobei das Lagerelement (106, 107) durch Presspassung fest mit dem ringförmigen Element (104) verbunden ist.

6. Verankerungselement (100) nach Anspruch 1 , wobei angrenzend an den Schaft (7) ein Kopf (6) vorgesehen ist, der in dem Aufnahmeteil (102) in einem Lagerelement (103) relativ zum dem stabförmigen Element drehbar gehalten ist.

7. Verankerungselement (100) nach einem der Ansprüche 3 bis 6, wobei das Lagerelement (106, 107) aus körperverträglichem Kunststoff, bevorzugt aus Polyethylen ausgeführt ist.

8. Verankerungselement (415) nach Anspruch 1, wobei angrenzend an den Schaft (412) ein Kopf (401) vorgesehen ist, der in dem Aufnahmeteil (408) so fixierbar ist, daß der Schaft (412) eine vorgegebene Winkelstellung relativ zu dem Aufnahmeteil (408) aufweist und wobei der Kopf (401) und der Schaft (412) separate Teile sind, die drehbar miteinander verbunden sind.

9. Verankerungselement (420) nach Anspruch 1, wobei angrenzend an den Schaft (412) ein Kopf vorgesehen ist, der mit dem Aufnahmeteil (421) fest verbunden ist bzw. ein integraler Bestandteil desselben ist und wobei der Kopf und der Schaft (412) separate Teile sind, die drehbar miteinander verbunden sind.

10. Verankerungselement (100, 415, 420) nach einem der Ansprüche 1 bis 9, wobei ein Anschlag (149, 150, 406) zum Begrenzen der Drehbewegung vorgesehen ist.

11. Verankerungselement nach einem der Ansprüche 1 bis 10, wobei der in dem Knochen oder Wirbel zu verankernde Schaft (7, 412) ein Knochengewinde aufweist.

12. Stabilisierungseinrichtung (200) mit wenigstens zwei Verankerungselementen (202, 202') und einem diese verbindenden stabförmigen Element (201), wobei
wenigstens ein Verankerungselement nach einem der Ansprüche 1 bis 11 ausgebildet ist.

## Claims

1. An anchoring element (100, 415, 420) for anchoring a rod-shaped element (21, 407) in a bone or a vertebra, the anchoring element comprising
a shaft (7, 412) to be anchored in the bone or the vertebra,
a receiving part (102, 408, 421) connected to the shaft (7, 412) for receiving the rod-shaped element (21, 407), and
a fixation device (109, 410) for fixing the rod-shaped element (21, 407) in the receiving part (102, 408, 421),
**characterized in that**, in the fixed condition of the rod-shaped element (21, 407) the shaft (7, 412) is rotatably connected to the rod-shaped element (21, 407) via the receiving part (102, 408, 421), and
a guide surface (152, 406) is provided, which limits the relative movement between the shaft (7, 412) and the rod-shaped element (21, 407) to one degree of rotational freedom.

2. The anchoring element (100) of claim 1, wherein the shaft (7) is fixedly connected to the receiving part (102) and the rod-shaped element (21) is rotatably held in a mounting (108) in the receiving part (102).

3. The anchoring element (100) of claim 2, wherein the mounting (108) is rotatably mounted in a bearing element (106, 107).

4. The anchoring element (100) of claim 3 being configured as a polyaxial screw with a bone anchoring element (2) comprising the shaft (7) and a spherical segment-shaped head (6), wherein,
a spherical seat (121) is provided adjacent to one end of the receiving part (102) for receiving the spherical segment-shaped head (6) of the bone anchoring element (2),
two recesses (140) opposite to each other are provided adjacent to the other end of the receiving part (102) by means of which two free legs (114, 115) are formed,
at the insides of the legs (114, 115) a thread (122) is provided, in which a ring-shaped element (104) can be screwed-in, by which pressure can be exerted directly or indirectly onto a pressure element (103), which in turn transfers said pressure to the head (6) of the bone anchoring element (2), so that the bone anchoring element (2) is fixed in a predetermined angle position relative to the receiving part (102), wherein
the bearing element (106, 107) resides inside the ring-shaped element (104).

5. The anchoring element (100) of claim 4, wherein the bearing element (106, 107) is firmly connected to the ring-shaped element (104) by means of press-fitting.

6. The anchoring element (100) of claim 1, wherein adjacent to shaft (7) a head (6) is provided which is rotatably held relative to the rod-shaped element in a bearing element (103) in the receiving part (102).

7. The anchoring element (100) of one of claims 3 to 6, wherein the bearing element (106, 107) is made from a body-compatible plastic material, preferably from polyethylene.

8. The anchoring element (415) of claim 1, wherein adjacent to the shaft (412) a head (401) is provided which is fixable in the receiving part (408) in such a manner that the shaft (412) has a predetermined angle position relative to the receiving part (408) and wherein the head (401) and the shaft (412) are separate parts which are rotatably connected to each other.

9. The anchoring element (420) of claim 1, wherein adjacent to the shaft (412) a head is provided which is fixedly connected to the receiving part (421) or which is an integral component thereof and wherein the head and the shaft (412) are separate parts which are rotatably connected to each other.

10. The anchoring element (100, 415, 420) of one of claims 1 to 9, wherein a stop (149, 150, 406) is provided for limiting the rotating movement.

11. The anchoring element of one of claims 1 to 10, wherein the shaft (7, 412) to be anchored in the bone or the vertebra comprises a bone thread.

12. A stabilization device (200) comprising at least two anchoring elements (202, 202') and a rod-shaped element (201) connecting the anchoring elements, wherein at least one anchoring element is configured according to one of claims 1 to 11.

## Revendications

1. Organe de fixation (100, 415, 420) destiné à fixer un élément en forme de tige (21, 407) dans un os ou une vertèbre, comportant
une tige (7, 412) à fixer dans l'os ou la vertèbre,
un élément de réception (102, 408, 421) relié à la tige (7, 412) et destiné à recevoir l'élément en forme de tige (21, 407), et,
un dispositif d'immobilisation (109, 410) destiné à immobiliser l'élément en forme de tige (21, 407) dans l'élément de réception (102, 408, 421),
**caractérisé en ce que**, dans la position immobilisée de l'élément en forme de tige (21, 407), la tige (7, 412) est reliée de manière rotative à l'élément en forme de tige (21, 407) par l'intermédiaire de l'élément de réception (102, 408, 421), et
il est prévu une surface de guidage (152, 406), par laquelle le mouvement relatif entre la tige (7, 412) et l'élément en forme de tige (21, 407) est limité à un degré de liberté en rotation.

2. Organe de fixation (100) selon la revendication 1, dans lequel la tige (7) est reliée de manière fixe à l'élément de réception (102), et l'élément en forme de tige (21) est maintenu de manière à pouvoir tourner dans une douille (108) dans l'élément de réception (102).

3. Organe de fixation (100) selon la revendication 2, dans lequel la douille (108) est montée rotative dans un palier (106, 107).

4. Organe de fixation (100) selon la revendication 3, réalisé sous forme de vis polyaxiale comportant un élément de fixation à l'os (2) formé par la tige (7) et une tête (6) en forme de segment sphérique, dans lequel
un siège (121) sphérique, adjacent à une extrémité de l'élément de réception (102), est prévu pour recevoir la tête (6) en forme de segment sphérique de l'élément de fixation à l'os (2),
il est prévu deux évidements (140) face à face adjacents à l'autre extrémité de l'élément de réception (102), par l'intermédiaire desquels sont formés deux flancs (114, 115) libres,
sur les faces intérieures des flancs (114, 115) libres est prévu un filetage (122), dans lequel peut se visser un élément annulaire (104) permettant d'appliquer directement ou indirectement une pression sur un élément de pression (103) qui, pour sa part, transmet cette pression sur la tête (6) de l'élément de fixation à l'os (2), de telle sorte que ledit élément de fixation à l'os (2) est immobilisé dans une position angulaire prédéfinie par rapport à l'élément de réception (102),
le palier (106, 107) étant logé à l'intérieur de l'élément annulaire (104).

5. Organe de fixation (100) selon la revendication 4, dans lequel le palier (106, 107) est relié de manière fixe à l'élément annulaire (104) par un ajustage serré.

6. Organe de fixation (100) selon la revendication 1, dans lequel est prévue une tête (6), qui est adjacente à la tige (7) et est maintenue dans l'élément de réception (102) de manière à pouvoir tourner par rapport à l'élément en forme de tige dans un palier (103).

7. Organe de fixation (100) selon l'une quelconque des revendications 3 à 6, dans lequel le palier (106, 107) est réalisé dans une matière plastique biocompatible, de préférence en polyéthylène.

8. Organe de fixation (415) selon la revendication 1, dans lequel est prévue une tête (401), qui est adjacente à la tige (412) et qui peut être immobilisée dans l'élément de réception (408) de telle sorte que la tige (412) présente une position angulaire prédéfinie par rapport à l'élément de réception (408), et dans lequel la tête (401) et la tige (412) sont des pièces séparées qui sont reliées de manière rotative l'une à l'autre.

9. Organe de fixation (420) selon la revendication 1, dans lequel est prévue une tête, qui est adjacente à la tige (412) et qui est reliée de manière fixe à l'élément de réception (421) ou est une pièce intégrale de celui-ci, et dans lequel la tête et la tige (412) sont des pièces séparées qui sont reliées de manière rotative l'une à l'autre.

10. Organe de fixation (100, 415, 420) selon l'une quelconque des revendications 1 à 9, dans lequel est prévue une butée (149, 150, 406) destinée à limiter le mouvement de rotation.

11. Organe de fixation selon l'une quelconque des revendications 1 à 10, dans lequel la tige (7, 412), destinée à être fixée dans l'os ou la vertèbre, comporte un filetage pour os.

12. Dispositif de stabilisation (200) comportant au moins deux organes de fixation (202, 202') et un élément en forme de tige (201) reliant ceux-ci,
dans lequel au moins un organe de fixation est réalisé selon l'une quelconque des revendications 1 à 11.
